# EUROPEAN PATENT SPECIFICATION

(11) **EP 0 560 918 B1**
(45) Date of publication and mention of the grant of the patent: **23.06.1999**
(21) Application number: 92902338.0
(22) Date of filing: 06.12.1991
(51) Int. Cl.: A61M 1/00

(54) **ABSORBENT-CONTAINING DRAINAGE BAG AND METHOD OF USE**
ABSORBENS ENTHALTENDER DRAINAGESACK SOWIE VERFAHREN ZUR ANWENDUNG
POCHE DE DRAINAGE CONTENANT UN PRODUIT ABSORBANT ET PROCEDE D'UTILISATION

(30) Priority: 07.12.1990 US 624115
(43) Date of publication of application: 22.09.1993
(73) Proprietor: Eastman, Dianne, Kensington California 94707 (US)
(72) Inventor: Eastman, Dianne, Kensington California 94707 (US)
(74) Representative: Findlay, Alice Rosemary
(86) International application number: US9109314
(87) International publication number: WO9210220

(56) References cited:
- FR-A- 2 380 069
- US-A- 4 314 558
- US-A- 4 713 067
- US-A- 4 790 834
- US-A- 4 820 291
- US-A- 4 840 625
- US-A- 4 994 051

## Description

The present invention relates to a drainage bag for biological fluids, particularly one suitable for storing urine from a catheterized or ostomy patient.

The urine from a catheterized or ostomy patient is stored in a urinary drainage bag (typically 2000cc capacity) connected by a tubing to one end of a catheter which communicates at its other end with a patient's urinary tract. Typically, the bag is hung on the bed. It includes a removable cap at its bottom for emptying urine at periodic intervals, typically every twelve hours, for durations as long as a number of weeks or more. The bacteria builds up in the warm stale urine remaining in the bag.

It has been found that the bacteria swim in the bag and the tube connected to the catheter. Moreover, on drainage, the bacteria-containing urine typically splashes to cause contamination in the room. This is particularly dangerous when a person is on antibiotics because a resistant strain can be carried to other patients through contamination on the floor and on the health care worker. It has been reported that over ninety percent of the patients who are on catheters and on drainage bags contract severe urinary tract infections and that approximately 50,000 patients per year die of urinary tract infections. At a minimum, such infections are uncomfortable and costly to the patient.

There is a need for a disposable drainage bag unit, particularly one for collecting urine from a catheterized or ostomy patient, which significantly reduces urinary tract infections.

U.S. Patent 4,700,714 discloses a urine collection device for wearing under clothes for collecting a urine specimen. Contained within the collection device is a dry compressed cellulose sponge for precisely controlling the quantity of urine retained by the bag. The unit specifically is made in a small capacity (up to 23cc) and would not be useful as a drainage bag.

U.S. Patent 4,415,388 discloses particulate absorbents of water-insoluble water-swellable polymer which are immobilized in a liquid polyhydroxy organic compound. The composition is applied to a surface and the liquid film solidifies. The product is disclosed to provide absorbency to bedpans, sanitary products, diapers, incontinent pads and the like.

U.S. Patent 4,534,767 discloses protective sealing compositions in the form of molded rings or sheets which include a gelled mixture of water-absorbing particulate hydrocolloid gum and non-toxic liquid polyhydroxy alcohol.

Super-absorbent polymers in the form of free-flowing powder have been recently developed which can absorb hundreds of time their own weight in water. In the soil, the powder becomes a gel which absorbs the water and holds nutrients for plant roots. One such polymer is sold by Broadleaf Industries, Inc. of San Diego, California under the trademark P4. It is a high molecular weight, cross-linked polyacrylamide. On absorption, the granules, which may be of a sugar-like texture, expand into clear, translucent pieces of gel, typically ranging from 0.64 to 0.95 cm (1/4 to 3/8 inch). There is no suggestion in the literature that such product would be useful for medical applications.

French Patent 2380069 and equivalent U.S. Patent 4179367 describes the use of a cross-linking polymer to alter the consistency of excretion products to a soft, flowable gel. A hydrophillic adsorption agent may be added to convert the products to a stiff, non-flowable, structured gel. In either case liquid slopping in a discharge collection bag is prevented.

U.S. Patent No. 4314558 describes a surgical drainage bag suitable for receiving body fluids and wastes. The bag contains absorbent particles either dusted on the interior surface or carried on a film matrix. The particles may be of a superabsorbent cross-linked material capable of absorbing many times its own weight of fluid.

A drainage device for collecting biological fluid draining therein from a patient through a drainage tubing, in accordance with the invention, comprises a flexible impermeable drainage bag containing therein a quantity of free-flowing, water-absorbing, water-swellable, water-insoluble, gel-forming polymer particles of a high molecular weight cross-linked acrylamide polymer, the polymer particles being capable of absorbing and immobilizing biological fluids, characterised in that the bag has an enclosed interior compartment and a port communicating with the enclosed interior compartment, in that the polymer particles are capable of absorbing at least about 500 times their weight of biological fluid to form expanded gel pieces for encapsulating and immobilizing toxic bacteria present in the biological fluids introduced into the enclosed interior compartment via the port, and in that a predetermined quantity of polymer particles is provided in the interior compartment, the predetermined quantity, relative to a quantity of biological fluid sufficient to substantially fill the expanded drainage bag, being at least 0.25 gram per 100 ml of biological fluid that enters into the enclosed interior compartment via the port to cause the polymer particles to expand so as to form a mass of self-supporting expanded gel pieces substantially throughout the drainage bag, whereby the expanded gel pieces are capable of encapsulating and substantially immobilizing the toxic bacteria present in the enclosed interior compartment to prevent any substantial movement of the toxic bacteria out of the enclosed interior compartment via the port and to create an inert environment in the enclosed interior compartment which is non-viable for the encapsulated and immobilized toxic bacteria.

The invention also provides a corresponding method of collecting biological fluid draining from a patient into a drainage bag.

In a preferred embodiment, the drainage device is provided for collecting biological fluid, such as urine, draining from a catheter connected to a patient. Prior to being filled with the biological fluid, the drainage bag contains free-flowing, water-absorbing, water-swellable, water-insoluble, gel-forming polymer particles. Such particles, preferably cross-linked polyacrylamides, are capable of absorbing many times their own weight of water to form expanded gel pieces. The quantity of the particles are sufficient to cause them to be expanded in the drainage bag to form a mass of self-supporting gel pieces substantially throughout the drainage bag. Such gel pieces are capable of substantially immobilizing toxic bacteria present in the drainage bag to prevent substantial movement out of the drainage bag and through the tubing. The drainage bag has a volume capacity of at least 100cc, typically on the order of 1,000 to 2,000cc for urinary drainage. The bag may also be used for wound drainage.

### BRIEF DESCRIPTION OF THE DRAWINGS

Figure 1 illustrates a drainage bag in accordance with the invention attached to a catheter prior to filling.
Figure 2 illustrates a drainage bag mostly filled with biological fluid and showing gel piece formation.
Figure 3 is a cross-section of Figure 2 taken at the line 3-3 illustrating the gel pieces according to the invention.

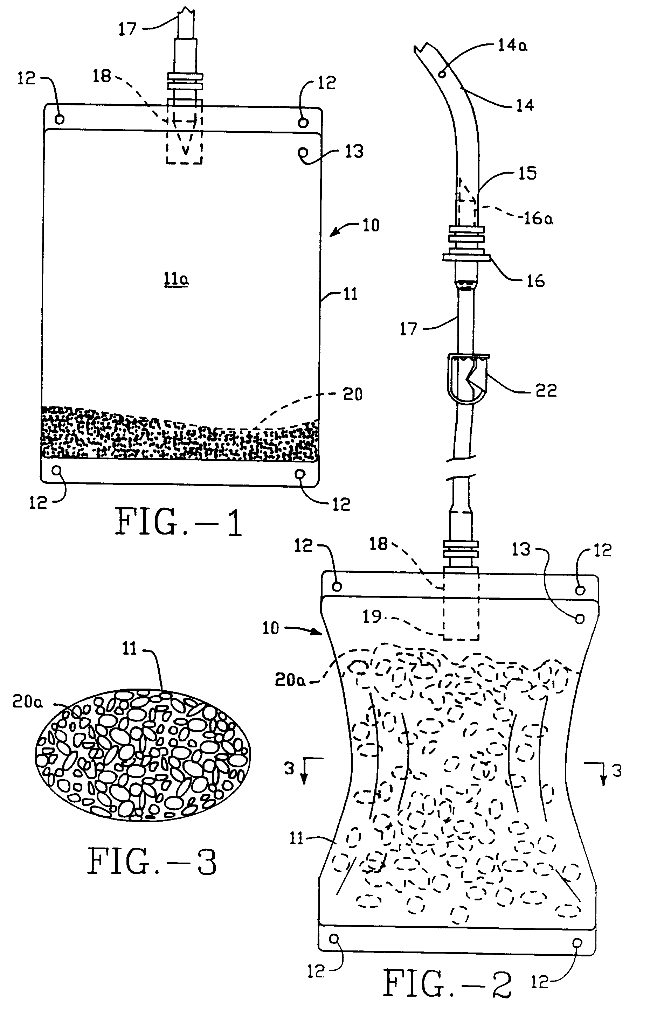

Referring to Figure 1, a drainage device generally illustrated by the number 10 is illustrated for collecting biological fluid draining from a patient. Drainage device 10 includes a flexible, impermeable drainage bag 11 suitably formed of a conventional transparent flexible plastic material in the shape of a typical ostomy drainage bag including connecting rectangular sidewalls sealed at their adjacent edges to substantially flatten when empty and define an interior chamber 11a upon filling. Bag 11 includes eyes 12 in the top and bottom sealed edges for stringing to an adjustable hanging hook which typically sits on the patient's bed. Bag 11 includes a bacteria air vent 13 near its top edge to permit the bag to breathe and promote drainage. For a catheterized patient, the drainage bag typically is connected subcutaneously to the patient's urinary tract by a conventional catheter 14 suitably in the form of a flexible 30.5 cm (one-foot) long rubber tube having openings 14a communicating with the urinary tract. Only a short stub of the catheter projects from the patient's skin. At the other end of catheter 14 is an enlarged cup 15 forming a female opening suitable for receiving and retaining male end of tubing connected to the drainage bag for forming a detachable seal.

As illustrated, the catheter connection is formed by a fitting 16 terminating in a rigid plastic finger 16a which is tightly seated within cup 15 to form a fluid seal. Fitting 16 is attached to the distal end of flexible plastic tubing 17 which is either permanently or detachably connected to an inlet port 18 of drainage bag 11 which communicates with interior chamber 11a.

As illustrated, port 18 is detachably coupled to tubing 17. This eliminates breaking the sterile connection with the catheter during bag replacement. Port 18 includes a relatively rigid connector tube 19 which projects at one end into the interior of chamber 11a and at the other end outwardly from drainage bag 11 for receiving the proximal end of tubing 17 in a detachable sealing engagement. Tubing 17 typically is about 61 cm (two feet) long and is formed of a flexible plastic and includes a rigid fitting which detachably locks and seals with the interior of connection tube 19 to form a detachable coupling, by a snap-lock, tapered force-fit, screw-fit, or the like. Connection tube 19 includes a one-way valve passing liquid only in the direction of the bag.

In an alternative embodiment, not shown, the proximal end of tubing 17 is permanently affixed to coupling 19 as by an adhesive or heat welding.

In another alternative embodiment, tubing 17 is connected to the outlet of an ostomy connection, rather than to a catheter.

Drainage bag 11 typically has a capacity of 1,000-2,000cc or more when used as a urinary drainage bag. Similar configurations can be used for receiving fluids from wound drainage, but the volume of the drainage bag in that instance would be less, but still at least about 100cc.

Polymer particles 20, preferably in powder form, are disposed in bag drainage 11 prior to use. Such particles are free-flowing, water-absorbing, water-swellable, water-insoluble, gel-forming polymer materials. While particles 20 are preferably in a dry sterile form prior to use, they may also be partially dehydrated prior to use. They are capable of absorbing many times their own weight of water to form expanded gel pieces, typically in irregular shapes of 0.25 to 0.75 cm (0.1 to 0.3 inch) nominal diameter. The quantity of the polymer particles is sufficient to cause them to expand in the drainage bag and form a mass of such self-supporting gel pieces substantially throughout the drainage bag when the bag is filled with biological fluid.

Effective polymers are cross-linked polyacrylamides. As used herein, the term polyacrylamide includes polymers and copolymers of acrylamide. The copolymers include acrylamide-potassium acrylate and acrylamide acrylate. Other polymers may be employed such as potassium propenoate-propenamide copolymer and various grafted copolymers.

A preferred form of polymer for particles 20 is a cross-linked polyacrylamide sold by Broadleaf Industries, Inc. of San Diego, California under the trademark P4. It is a fine flowable powder or granular solid capable of absorbing over 500 times its own weight in water. Moreover, it is inert to bacteria and does not present a viable environment for the growth of such bacteria. This is contrasted with less-preferred cellulose-grafted absorbent polymers or sponges. Using the P4 product, about 5-10gm, preferably about 7.5gm, provides a suitable final gel piece mass for a standard 2000cc drainage bag.

Other polymers which may be used include cross-linked polyacrylamide copolymers such as sold under the brand name Agrosoke by Grosoke International of Fort Worth, Texas, potassium proteonate-propenamide copolymers such as sold under the name Viterra Plants-gel by Nepera Chemical Co. of Harriman, New York. Starch grafted copolymers can be used, but they are not as desirable as the foregoing polymers since they can biodegrade. It is advantageous for the particles to gel in a time which would permit the substantially complete absorbance of water into gel particles at the time of breaking seal to replace the bag. To assure that the last biological fluid received by the bag is absorbed into the gel pieces, it is preferable for the particles to absorb substantially all of the liquid within one hour, preferably within 20 minutes.

An important property of the polymer particles is that they expand in the presence of the urine to self-supporting granules, substantially preventing continuous liquid paths of bacteria from the mass of gel granules. It has been found that the expanded gel granules are capable of substantially immobilizing bacteria when present in the drainage bag to prevent substantial movement of the toxic bacteria out of the drainage bag and into the biological fluid in the tubing. Such bacteria tend to enter the drainage bag when the seal between the drainage bag and catheter is broken during the course of connecting a new drainage bag after disposal of the prior full drainage bag. The seal breakage can occur either at the connection of the tubing to the catheter or the tubing to the drainage bag, depending upon whether the drainage bag is permanently affixed to the tubing as will be described below.

Another advantage of the particles of the present invention is that when they form the mass of self-supporting gel pieces, there is no splashing liquid in the drainage bag at the time of bag replacement. It is well recognized that during replacement, the splash of biological fluid (e.g. urine) is a very dangerous source of contamination, both within the bag and when splashing into the room.

Referring to Figures 2 and 3, the bag of Figure 1 is illustrated mostly filled with biological fluid illustrating gel pieces 20a. The previously flat bag expands into a pillow shape as in a conventional drainage bag, but it is firm, being substantially completely filled with a mass of gel granules 20a.

It is preferable to include a conventional bactericide within the bag.

Use of the drainage device 10 first will be described with respect to the embodiment of Figure 1 in which the tubing 17 is detachably coupled to connector 19. Catheter 14 is implanted into the urinary tract. The bag hangs with tubing 17 in a vertical upright position with the bag below the level of the patient. A valve in the form of a biased clamp 22 on the tubing on the catheter is initially closed. Then, it is opened for drainage of the patient's urine (or other biological fluid) through catheter 14 into bag 11 which contains polymer particles 20. Drainage continues until the bag is filled or the bag is earlier replaced by an empty one. Prior to replacement, clamp 22 is closed and tubing 17 is removed from connector 19. At this time, there is substantially no free-flowing fluid in the drainage bag since substantially all of the biological fluid is retained within the mass of gel particles. Then, the drainage bag is disposed of and another drainage bag is connected to the tubing 17 and the procedure is done again.

In the alternative where tubing 17 is permanently sealed to tubing 17, bag 11 is detached by decoupling the connection between finger 16a and cup 15. In that instance, clamp 22 is clamped at the time of disconnection.

## Claims

1. A drainage device (10) for collecting biological fluid draining therein from a patient through a drainage tubing (17) comprising a flexible impermeable drainage bag (11) containing therein a quantity of free-flowing, water-absorbing, water-swellable, water-insoluble, gel-forming polymer particles (20) of a high molecular weight cross-linked acrylamide polymer, the polymer particles (20) being capable of absorbing and immobilizing biological fluids, characterised in that the bag (11) has an enclosed interior compartment and a port (18) communicating with the enclosed interior compartment, in that the polymer particles (20) are capable of absorbing at least about 500 times their weight of biological fluid to form expanded gel pieces (20a) for encapsulating and immobilizing toxic bacteria present in the biological fluids introduced into the enclosed interior compartment via the port (18), and in that a predetermined quantity of polymer particles (20) is provided in the interior compartment, the predetermined quantity, relative to a quantity of biological fluid sufficient to substantially fill the expanded drainage bag (11a), being at least 0.25 gram per 100 ml of biological fluid that enters into the enclosed interior compartment via the port (18) to cause the polymer particles (20) to expand so as to form a mass of self-supporting expanded gel pieces (20a) substantially throughout the drainage bag (11a), whereby the expanded gel pieces (20a) are capable of encapsulating and substantially immobilizing the toxic bacteria present in the enclosed interior compartment to prevent any substantial movement of the toxic bacteria out of the enclosed interior compartment via the port (18) and to create an inert environment in the enclosed interior compartment which is non-viable for the encapsulated and immobilized toxic bacteria.

2. A drainage device as claimed in Claim 1, wherein the biological fluid is urine.

3. A drainage device as claimed in either Claim 1 or 2, wherein the drainage tubing (17) is coupled at one end to the port (18) communicating with the enclosed interior compartment of the drainage bag (11) and is adapted for connection at its other end to a catheter (14).

4. A drainage device as claimed in Claim 3, wherein the drainage bag (11) is non-detachably coupled to the tubing (17).

5. A drainage device as claimed in either Claim 3 or Claim 4, wherein the drainage tubing (17) is detachably coupled to the catheter (14).

6. A drainage device as claimed in any preceding Claim, wherein the polymer particles (20) are formed of a polymer comprising an acrylamide-acrylate copolymer.

7. A drainage device as claimed in any preceding Claim, wherein the enclosed interior compartment of the drainage bag (11) has a capacity of at least 100 ml.

8. A method of collecting biological fluid draining from a patient into a drainage bag (11) comprising the steps of flowing the biological fluid from a patient's body cavity through a catheter (14) and into the drainage bag (11) containing a quantity of free-flowing, water-absorbing, water-swellable, water-insoluble, gel-forming polymer particles (20) of a high molecular weight cross-linked acrylamide polymer, and mixing the biological fluid with the particles (20), characterised in that the polymer particles (20) are capable of absorbing at least about 500 times their weight of biological fluid to form expanded gel particles (20a) for encapsulating and immobilizing toxic bacteria present in the biological fluid, in that a predetermined quantity of polymer particles (20) is provided, the predetermined quantity, relative to a quantity of biological fluid sufficient to substantially fill the expanded drainage bag (11a), being at least about 0.25 gram per about 100 ml of biological fluid that enters into the drainage bag (11), and in that the method includes mixing the biological fluid with the predetermined quantity of polymer particles (20) so as to form a mass of self-supporting expanded gel pieces (20a) substantially throughout the drainage bag encapsulating and immobilizing the toxic bacteria present in the biological fluid with the self-supportng expanded gel pieces (20a) to prevent any substantial movement of the toxic bacteria out of the drainage bag (11a) and into the catheter (14) and to create an inert environment in the drainage bag (11a) which is non-viable for the encapsulated and immobilized toxic bacteria.

9. A method as claimed in Claim 8, wherein the biological fluid comprises urine.

10. A method as claimed in either Claim 8 or 9, wherein the polymer particles (20) are formed of a polymer comprising an acrylamide-acrylate copolymer.

## Patentansprüche

1. Drainagevorrichtung (10) zum Auffangen eines biologischen Fluids, das von einem Patienten durch Drainageschläuche (17) abgelassen wird, aufweisend einen flexiblen undurchlässigen Drainagesack (11), der eine Menge an rieselfähigen, wasserabsorbierenden, wasserquellbaren, wasserunlöslichen, gelbildenden Polymerteilchen (20) aus einem vernetzten Acrylamidpolymer mit hohem Molekulargewicht enthält, wobei die Polymerteilchen (20) geeignet sind, biologische Fluide zu absorbieren und festzulegen,
**dadurch gekennzeichnet**,
daß der Sack (11) eine eingeschlossene innere Kammer und eine Einlaßöffnung (18) aufweist, die mit der eingeschlossenen inneren Kammer in Verbindung steht, daß die Polymerteilchen (20) geeignet sind, zumindest das etwa 500-Fache ihres Gewichts an biologischem Fluid zu absorbieren, so daß expandierte Gel-Teilchen (20a) gebildet werden, um toxische Bakterien, die in den biologischen Fluiden vorliegen, die über die Einlaßöffung (18) in die eingeschlossene innere Kammer eingeführt worden sind, zu umschließen und festzulegen, und daß eine bestimmte Menge an Polymerteilchen (20) in der inneren Kammer vorgesehen ist, wobei die bestimmte Menge an Polymerteilchen in bezug auf die Menge an biologischem Fluid ausreicht, um den ausgeweiteten Drainagesack (11a) im wesentlichen zu füllen, wobei zumindest 0,25 Gramm pro 100 ml des biologischen Fluids über die Einlaßöffnung (18) in die eingeschlossene innere Kammer eintritt, um zu bewirken, daß die Polymerteilchen (20) sich ausdehnen, so daß sie im wesentlichen in dem gesamten Drainagesack (11a) eine Masse aus sich selbst haltenden expandierten Gel-Teilchen (20a) bilden, wobei die expandierten Gel-Teilchen (20a) geeignet sind, die toxischen Bakterien, die in der eingeschlossenen inneren Kammer vorliegen, einzuschließen und im wesentlichen festzulegen, um jede signifikante Bewegung der toxischen Bakterien durch die Einlaßöffnung (18) aus der eingeschlossenen inneren Kammer hinaus zu verhindern und in der eingeschlossenen inneren Kammer eine inerte Umgebung zu erzeugen, in der die eingeschlossenen und festgehaltenen toxischen Bakterien nicht lebensfähig sind.

2. Drainagevorrichtung nach Anspruch 1, in der das biologische Fluid Urin ist.

3. Drainagevorrichtung nach Anspruch 1 oder 2, in der der Drainageschlauch (17) an einem Ende an die Einlaßöffnung (18) angeschlossen ist, die mit der eingeschlossenen inneren Kammer des Drainagesacks (11) in Verbindung steht, und geeignet ist, mit seinem anderen Ende mit einem Katheter (14) verbunden zu werden.

4. Drainagevorrichtung nach Anspruch 3, in der der Drainagesack (11) unlösbar an den Schlauch (17) angeschlossen ist.

5. Drainagevorrichtung nach Anspruch 3 oder Anspruch 4, in der der Drainageschlauch (17) lösbar an den Katheter (14) angeschlossen ist.

6. Drainagevorrichtung nach einem der vorhergehenden Ansprüche, in der die Polymerteilchen (20) aus einem Polymer gebildet sind, das ein Acrylamid-Acrylat-Copolymer umfaßt.

7. Drainagevorrichtung nach einem der vorhergehenden Ansprüche, in der die eingeschlossene innere Kammer des Drainagesacks (11) eine Kapazität von mindestens 100 ml hat.

8. Verfahren zum Auffangen eines biologischen Fluids, das von einem Patienten in einen Drainagesack (11) abgelassen wird, das die Schritte beinhaltet, daß man das biologische Fluid aus der Körperhöhle eines Patienten durch einen Katheter (14) und in den Drainagesack (11) fließen läßt, der eine Menge an rieselfähigen, wasserabsorbierenden, wasserquellbaren, wasserunlöslichen, gelbildenden Polymerteilchen (20) aus einem vernetzten Acrylamidpolymer mit hohem Molekulargewicht enthält, und das biologische Fluid mit den Teilchen (20) mischt,
**dadurch gekennzeichnet**,
daß die Polymerteilchen (20) geeignet sind, mindestens das etwa 500-Fache ihres Gewichts an biologischem Fluid zu absorbieren, so daß sie expandierte Gel-Teilchen (20a) bilden, um die toxischen Bakterien, die in dem biologischen Fluid vorliegen, einzuschließen und festzulegen, daß eine bestimmte Menge an Polymerteilchen (20) vorgesehen ist, wobei die bestimmte Menge in bezug auf eine Menge an biologischem Fluid ausreicht, um den expandierten Drainagesack (11a) im wesentlichen zu füllen, wobei zumindest etwa 0,25 Gramm pro etwa 100 ml des biologischen Fluids in den Drainagesack (11) eintritt, und daß das Verfahren das Mischen des biologischen Fluids mit der bestimmten Menge an Polymerteilchen (20) umfaßt, so daß im wesentlichen im gesamten Drainagesack eine Masse aus sich selbst haltenden expandierten Gel-Teilchen (20a) gebildet wird, die die toxischen Bakterien, die in dem biologischen Fluid vorliegen mit den sich selbst haltenden expandierten Gel-Teilchen (20a) einschließen und festlegen, um jede signifikante Bewegung der toxischen Bakterien aus dem Drainagesack (11a) und in den Katheter (14) zu verhindern, und in dem Drainagesack (11a) eine inerte Umgebung zu erzeugen, in der die eingeschlossenen und festgehaltenen toxischen Bakterien nicht lebensfähig sind.

9. Verfahren nach Anspruch 8, wobei das biologische Fluid Urin beinhaltet.

10. Verfahren nach Anspruch 8 oder Anspruch 9, wobei die Polymerteilchen (20) aus einem Polymer gebildet werden, das ein Acrylamid-Acrylat-Copolymer umfaßt.

## Revendications

1. Dispositif de drainage (10) pour recueillir un fluide biologique drainé à partir d'un patient par l'intermédiaire d'un tube de drainage (17) comprenant une poche de drainage imperméable flexible (11) renfermant une certaine quantité de particules de polymère (20) formant un gel, insolubles dans l'eau, gonflables à l'eau, absorbant l'eau et s'écoulent librement, formées d'un polymère d'acrylamide réticulé de masse moléculaire élevée, les particules de polymère (20) étant capables d'absorber et d'immobiliser des fluides biologiques, caractérisé en ce que la poche (11) a un compartiment intérieur clos et un orifica (18) communiquant avec le compartiment intérieur clos, en ce que les particules de polymère (20) sont capables d'absorber au moins environ 500 fois leur poids de fluide biologique pour former des fragments de gai expansé (20a) afin d'encapsuler et d'immobiliser les bactéries toxiques présentes dans les fluides biologiques introduite dans le compartiment intérieur clos par l'intermédiaire de l'orifice (18), et en ce qu'une quantité prédéterminée de particules de polymère (20) est prévue dons le compartiment intérieur, la quantité prédéterminée, par rapport à une quantité de fluide biologique suffisante pour remplir sensiblement la poche de drainage gonflée (11a), étant d'au moins 0,25 gramme pour 100 ml de fluide biologique entrant dans le compartiment intérieur clos par l'intermédiaire de l'orifice (18) pour provoquer l'expansion des particules de polymère (20) de façon à former une masse de fragments de gel expansés indépendants (20a) sensiblement dans toute la poche de drainage (11a), les fragments de gal expansés (20a) étant ainsi capables d'encapsuler et d'immobiliser sensiblement les bactéries toxiques présentes dans le compartiment intérieur clos afin d'empêcher sensiblement toute propagation des bactéries toxiques hors du compartiment intérieur clos par l'intermédiaire de l'orifice (18) et de créer un environnement inerte dans le compartiment intérieur clos qui est non viable pour las bactéries toxiques encapsulées et immobilisées.

2. Dispositif de drainage selon la revendication 1, dans lequel le fluide biologique est de l'urine.

3. Dispositif de drainage selon la revendication 1 ou 2, dans lequel le tube de drainage (17) est relié au niveau de l'une de ses extrémités à l'orifice (18) communiquant avec le compartiment intérieur clos de la poche de drainage (11) et est adapté pour être relié au niveau de son autre extrémité à un cathéter (14).

4. Dispositif de drainage selon la revendication 3, dans lequel la poche de drainage (11) est reliée au tube (17) de façon non détachable.

5. Dispositif de drainage selon la revendication 3 ou la revendication 4, dans lequel le tube de drainage (17) est relié au cathéter (14) de façon détachable.

6. Dispositif de drainage selon l'une quelconque des revendications précédentes, dans lequel les particules de polymère (20) sont formées d'un polymère comprenant un copolymère acrylamide/acrylate.

7. Dispositif de drainage selon l'une quelconque des revendications précédentes, dans lequel le compartiment intérieur clos de la poche de drainage (11) a une capacité d'au moins 100 ml.

8. Procédé pour recueillir un fluide biologique drainé à partir d'un patient dans une poche de drainage (11), comprenant les étapes consistant à faire écouler le fluide biologique à partir d'une cavité corporelle du patient, par l'intermédiaire d'un cathéter (14), dans la poche de drainage (11) contenant une certaine quantité de particules de polymère (20) formant un gel, insolubles dans l'eau, gonflables à l'eau, absorbant l'eau et s'écoulent librement, formées d'un polymère d'acrylamide réticulé de masse moléculaire élevée, et à mélanger le fluide biologique avec les particules (20), caractérisé en ce que les particules de polymère (20) sont capables d'absorber au moins environ 500 fois leur poids de fluide biologique pour former des particules de gel expansées (20a) afin d'encapsuler et d'immobiliser les bactéries toxiques présentes dans le fluide biologique, en ce qu'une quantité prédéterminée de particules de polymère (20) est prévue, la quantité prédéterminée, par rapport à une quantité de fluide biologique suffisante pour remplir sensiblement la poche de drainage gonflée (11a), étant d'au moins environ 0,25 gramme pour environ 100 ml de fluide biologique entrant dans le poche de drainage (11), et en ce que le procédé comprend le mélange du fluide biologique avec la quantité prédéterminée de particules de polymère (20) de façon à former une masse de fragments de gel expansés indépendants (20a) sensiblement dans toute la poche de drainage encapsulant et immobilisant les bactéries toxiques présentes dans le fluide biologique avec les fragments de gel expansés indépendants (20a) afin d'empêcher sensiblement toute propagation des bactéries toxiques hors de la poche de drainage (11a) et dans le cathéter et de créer un environnement inerte dans la poche de drainage (11a) qui est non viable pour les bactéries toxiques encapsulées et immobilisées.

9. Procédé selon la revendication 8, dans lequel le fluide biologique comprend de l'urine.

10. Procédé selon le revendication 8 ou 9, dans lequel les particules de polymère (20) sont formées d'un polymère comprenant un copolymère acrylamide/acrylate.
